# EUROPÄISCHE PATENTSCHRIFT

(11) **EP 0 824 363 B1**
(45) Veröffentlichungstag und Bekanntmachung des Hinweises auf die Patenterteilung: **27.09.2000**
(21) Anmeldenummer: 95927722.9
(22) Anmeldetag: 25.07.1995
(51) Int. Cl.: A61L 2/08

(54) **VERFAHREN ZUR HERSTELLUNG VON ERZEUGNISSEN AUS POLYMERWERKSTOFFEN MIT MEDIKAMENTÖSER DEPOTWIRKUNG**
PROCESS FOR MANUFACTURING POLYMER PRODUCTS WITH MEDICAMENTOUS DEPOT EFFECT
PROCEDE DE FABRICATION DE PRODUITS EN MATERIAUX POLYMERES A EFFET RETARD MEDICAMENTEUX

(30) Priorität: 10.05.1995 DE 19517167
(43) Veröffentlichungstag der Anmeldung: 25.02.1998
(73) Patentinhaber: VERITAS GUMMIWERKE AG, 63571 Gelnhausen (DE)
(72) Erfinder: SCHUNK, Werner, D-99867 Gotha (DE); MERKMANN, Gerhard, D-99867 Gotha (DE); GIESSMANN, Konrad, D-99867 Gotha (DE); LUDWIG, Hans-Josef, D-63571 Gelnhausen (DE); MERTENS, Wilfried, D-63619 Bad Orb (DE)
(74) Vertreter: Grünecker, Kinkeldey, Stockmair & Schwanhäusser Anwaltssozietät
(86) Internationale Anmeldenummer: EP9502943
(87) Internationale Veröffentlichungsnummer: WO9635459

(56) Entgegenhaltungen:
- DD-A- 275 697
- DE-A- 3 741 342
- DE-A- 3 744 289
- PL-A- 128 392
- DATABASE WPI Section Ch, Week 9028 Derwent Publications Ltd., London, GB; Class A96, AN 90-210626 XP002015424 & DD,A,275 697 (VEB GUMMIW THURING) , 31.Januar 1990 in der Anmeldung erwähnt

## Beschreibung

Die Erfindung betrifft ein Verfahren zur Herstellung von Erzeugnissen aus Polymerwerkstoffen mit medikamentöser Depotwirkung, die in der Medizin in und am menschlichen Organismus eingesetzt werden können.

Die Herstellung von Erzeugnissen aus Polymerwerkstoffen für den medizinischen Einsatz mit Hilfe herkömmlicher Vernetzungsverfahren, d.h. durch Heißluft-, Dampf-, UHF- und LCM (liquid curing method)-Verfahren, ist bereits bekannt. Über diese Verfahren sind wegen der hohen thermischen Belastung Polymererzeugnisse mit medikamentöser Depotwirkug nicht herstellbar.

Ferner ist aus der Patentschrift DE 37 41 342 ein Verfahren zur Herstellung eines medizinischen Materials mit hoher Antithrombogenizität bekannt, das u.a. für Katheter und andere medizinische Geräte geeignet ist, die mit Geweben und fließendem Blut in Berührung kommen. Dabei kann das beschriebene Material durch eine Polymerisation eines Methacrylamid-Dervivats mit einer tertiären Aminogruppe /oder eines hydrophilen Monomers mit einer die Copolymerisation beschleunigenden Eigenschaft auf einem hochmolekolaren Polyolefin- oder Polyurethansubstrat hergestellt werden. Durch eine ionisierende Strahlung werden aktive Stellen auf dem Substrat erzeugt. Anschließend wird das aktivierte Substrat in eine Lösung eines Monomers eingetaucht. Die antithrombogene Wirkung des Material wird folglich durch das aufgepfropfte Monomer bedingt.

Aus dem Vorangehenden wird deutlich, daß bei dem bekannten Stand der Technik das antithrombogen wirkende Material nur oberflächlich an das Substrat fixiert wird. Folglich kann eine länger anhaltende, kontinuierliche und über das Konzentrationsgefälle steuerbare Abgabe der biologisch aktiven Substanz nicht gewährleistet werden. Ferner befinden sich in dieser Druckschrift keine Hinweise auf eine biologische Aktivität der erzeugten Produkte.

In der **DE-A-3744289** ist ein Verfahren zum Herstellen von Hydrogelverbänden aus synthetischen und natürlichen Polymeren auf Kunststoffbasis durch Polymerisation und Vernetzung in wäßriger Lösung unter Anwendung einer ionisierenden Strahlung beschrieben. Die Hydrogelverbände enthalten kein biologisch wirksames Medikament. Das Wirkprinzip eines Polymerwerkstoffes im Sinne einer kontinuierlichen Arzneimittelverabreichung ist nicht Gegenstand der DE-A-3744289. Die in der DE-A-3744289 zitierte polnische Patentschrift PL-A-128392 beschreibt ein Verfahren zum Darstellen von Verbänden, die im Unterschied zum Verfahren gemäß DE-A-3744289 ein Heilmittel enthalten. Bei diesem Verfahren wird auf die mechanische Unterlage des Verbandes nach oder vor deren Sättigung mit dem Heilmittel einer Schicht eines hydorphilen Gels gebracht. Das Gel wurde einer Strahlungspolymerisation ausgesetzt. Anschließend wird der Verband getrocknet und sterilisiert. Daraus ergibt sich, daß die Strahlungspolymerisation und der Sterilisierungsvorgang nacheinander durchzuführen sind.

Desweiteren ist in der DD 275697 ein Verfahren zur Herstellung von Schläuchen für den medizinischen Einsatz offenbart, die biologisch wirksame Polymerwerkstoffe enthalten. Zur Vernetzung werden β-Strahlen unter Verwendung von Metalloxiden, wie ZnO oder TiO_{2,} als Vernetzungsaktivatoren eingesetzt. Durch dieses Verfanren können die extrem hohen Temperaturbelastungen, die durch die herkömlichen Verfahren bedingt sind, zwar vermieden werden, es treten jedoch andere Nachteile auf. So sind z.B. zur Vernetzung relativ hohe Strahlendosen notwendig, die wiederum ein erhebliches Maß an Reaktionswärme erzeugen. Zur Ableitung der Reaktionwärme müssen die Schläuche daher vor der Vernetzung auf Stahldorne aufgezogen werden. Es werden bei der Vernetzung die Produkt zwar auch sterilisiert, da aber der Stahldorn nicht mit verpackt werden soll, muß nach der Konfektionierung und Verpackung nochmals ein gesonderter Sterilisationsprozeß durchgeführt werden, der einerseits die Herstellungskosten erhöht und andererseits den Erhalt der biologischen Aktivität über den Herstellungsprozeß hinweg gefährdet, denn es ist bekannt, daß hohe Wärme- und/oder Strahlungsbeslastungen die biologische Aktivität organischer Verbindungen beeinträchtigen. Darüber hinaus sind die als Aktivatoren eingesetzten Metalloxide ZnO und TiO₂ als toxisch und daher für den Körper nachteilig zu bewerten.

Der Erfindung liegt daher die Aufgabe zugrunde, ein Verfahren zur Herstellung von Erzeugnissen aus Polymerwerkstoffen mit medikamentöser Depotwirkung zur Verfügung zu stellen, mit welchem die aus dem Stand der Technik bekannten Nachteile überwunden werden, und welches kostengünstig durchgeführt werden kann.

Diese Aufgabe wird erfindungsgemäß durch ein Verfahren zur Herstellung von Polymerwerkstoffen mit medikamentöser Depotwirkung gelöst, mit folgenden Schrittten:
- Vermischen der Ausgangsmaterialien,
- Formen der Mischung in eine bestimmte Form,
- Einbringen der so hergestellten Form in eine Schutzpackung, und
- Vernetzen und Sterilisieren der Form in der Schutzpackung

Durch das erfindungsgemäße Verfahren wird es möglich, die Prozeßschritte der Vernetzung und der Sterilisierung zu verbinden und so das Verfahren wesentlich einfacher und schneller durchzuführen. Ferner hat sich gezeigt, daß durch Kombination der Prozeßschritte der Vernetzung und Sterilisierung die sonst auftretende zusätzliche Wärme- oder Strahlenbelastung zur Sterilisation vermieden wird. Auf diese Weise kann sichergestellt werden, daß die biologische Aktivität der Erzeugnisses in voller Höhe erhalten bleibt und insbesondere während des Herstellungsprozesses nicht gefährdet ist. Des weiteren kann das erfindungsgemäße Verfahren kostengünstig durchgeführt werden.

Es hat sich dabei als vorteilhaft erwiesen, wenn das Vernetzen und Sterilisieren durch Elektronenbestrahlung erfolgt, wobei Strahlendosen von 10 bis 120 KGy, vorzugsweise 20 bis 66 KGy, verwendet sind.

Die β-Strahlung bewirkt sowohl die Vernetzung des Materials als auch dessen Sterilisierung. Dabei haben sich die angegebenen Strahlendosen als besonders bevorzugte Werte erwiesen, bei denen die Entstehung größerer Wärmenmengen verhindert werden kann und die Eigenschaften des Erzeugnisses nicht nachteilig beeinflußt werden.

Dem Polymerwerkstoff können je nach Notwendigkeit Substanzen zugegeben werden, die als Aktivatoren und Vernetzer wirken, um die Strahlendosis zu verringern. Dabei sind als aktivierende und vernetzende Substanz insbesondere Acrylate bevorzugt. Besonders bevorzugt ist die Zugabe von Trimethylolpropantrimethacrylat.

Die Zugabe dieser Stoffe, die gleichzeitig als Aktivatoren und auch als Vernetzer bei der Strahlenvernetzung wirken, ermöglicht eine hohe und gleichmäßige Vernetzungausbeute über das gesamte Volumen des Polymermaterials mit medikamentöser Depotwirkung und zugleich wird eine Vernetzung bei niedrigen Strahlendosen garantiert.

Methacrylate und deren Derivate haben sich zur Vernetzung des Basispolymers für biologisch aktive Materialen als besonders geeignet erwiesen, da sie weder die im Basispolymer enthaltene biologisch aktive Substanz in ihrer Aktivität beeinflussen, noch die aktive Substanz auf oder im Basispolymer fixieren und damit ihre gewollte Beweglichkeit zur Abgabe an vorbeifließende Medien behindern.

Ferner kann vorgesehen sein, daß eine biologisch aktive Substanz mit dem Polymerwerkstoff vermischt wird. Dabei kann die biologisch aktive Substanz bereits vor dem Vermischen mit dem Polymerwerkstoff an eine anorganische oder organische Trägersubstanz gebunden werden, z.B. an Molsiebe und/oder Schichtsilikate.

Durch das Einbringen einer biologischen Substanz in den Polymerwerkstoff kann eine dauerhafte Einlagerung derselben erzielt werden. Hierbei dient vorzugsweise eine Trägersubstanz als ein Depot und gestattet im Einsatzfall ihre kontinuierliche Abgabe an die angrenzenden flüssigen Medien, je nach dem herrschenden Konzentrationsgefälle.

Um die bei der Vernetzung auftretenden schädlichen Wärmemengen noch weiter zu verringern, ist es bevorzugt, den geformten Polymerwerkstoff nach dem Einbringen in die Schutzhülle und vor dem Vernetzen und Sterilisieren in eine wärmeleitende Form zu legen. Durch diese wärmeleitende Form wird die entstehende geringe Reaktionswärme abgeleitet und somit kann ein Wärmestau verhindert werden.

Weitere bevorzugte Ausführungsformen sind in den abhängigen Ansprüchen beschrieben.

Als Ausgangsmaterialen für Polymerwerkstoffe haben sich folgende Mischungsingredienzien als besonders geeignet erwiesen:

Naturkautschuk, Kreide, Vernetzungsaktivator, Molsieb-Medikament-Addukt oder Siliconkautschuk, Kreide, Molsieb-Medikament-Addukt.

Mit diesen Zusammensetzungen ist möglich, auf die als toxisch zu bewertenden Metalloxide ZnO und TiO₂ zu verzichten, Nebenreaktionen der Zusatzstoffe mit der biologisch aktiven Substanz unter Bestrahlung zu vermeiden und toxische Aktivitäten im Anwendungsfall durch weitere Zusatzstoffe auszuschließen. Ferner kann durch die Polymerauswahl bzw. deren Vorbehandlung und die erhöhte Dosierung der toxisch unbedenklichen Kreide als Streckmittel die notwendige Standfestigkeit und Verarbeitbarkeit auch ohne Fließ- und Stabilisierungshilfen, wie Stearinsäure und Faktis, erreicht werden.

Die erfindungsgemäße Mischung weist eine ausreichende Standfestigkeit auf, so daß auf den bisher üblichen Stahldorn als Stütze verzichtet werden kann.

Im folgenden wird die Erfindung anhand von bevorzugten Ausführungsbeispielen beschrieben:

Zwei bevorzugte Polymerwerkstoffe mit medikamentöser Depotwirkung wurden nach dem erfindungsgemäßen Verfahren hergestellt. Andere Zusammensetzungen werden damit nicht ausgeschlossen.

Folgende Zusammensetzung der Ausgangsmaterialien wurden verwendet:

| | Menge (Gew.%) |
|---|---|
| Naturkautschuk | 37,17 |
| Kreide | 46,47 |
| Vernetzungsaktivator¹⁾ | 1,49 |
| Molsieb/Medikament-Addukt²⁾ | 14,87 |
| | |
| und | |
| | |
| Siliconkautschuk | 52,6 |
| Kreide | 31,6 |
| Molsieb/Medikament-Addukt²) | 15,8 |

| | |
|---|---|
| ¹⁾ Trimethylolpropantrimethacrylat | |
| ²⁾ Molsieb 13X / Pentosanpolysulfat | |

Nach dem Vermischen der Ausgangsmaterialen durch herkömmliche Mischverfahren wurde die Mischung durch Extrudieren in eine gewünschte Form geformt, z.B. zu einem Schlauch.

Nach dem Beenden des Formens der Mischung wurde das geformte Polymermaterial in eine Schutzverpackung aus einer Polyamidfolie eingeschweißt. Diese frühzeitige Verpackung ist möglich, da die verwendete Mischung ausreichend standfest ist und daher die Form beibehält. In dieser Verpackung kann das Erzeugnis nach Fertigstellung verKauft und bis zum Einsatz gelagert werden. Anschließend wurde das verpackte Erzeugnis in eine Metallform aus z.B. Aluminium oder Kupfer eingelegt. Diese Metallform unterstützt die Wärmeableitung während der nachfolgenden Prozeßschritte.

Das in der Polyamid-Folie eingeschweißte Polymermaterial wurde mit β-Strahlen bestrahlt. Durch diese Elektronenbestrahlung wurde gleichzeitig das Vernetzen und Sterilisieren des Polymermaterials erzielt. Das Material wird folglich in einem Prozeßschritt fertiggestellt, und der im Stand der Technik notwendige weitere Sterilisierungsscnritt konnte entfallen.

Die bei den genannten Beispielen eingesetzte Strahlendosis betrug 33 KGy bzw. 66 KGy bei einer Bestrahlungsdauer von 20 sec.

Anschließend wurde ein Nachweis der biologischen Aktivität wie folgt durchgeführt:

Eine definierte Menge an Polymerwerkstoff mit medikamentöser Depotwirkung wurde in 4 ml Humanzitratplasma bei 37°C inkubiert. In Abhängigkeit der Inkubationszeit wurde das Ausmaß der Thrombinzeit bestimmt.

Die Ergebnisse sind in der nachfolgenden Tabelle dargestellt.

| | Thrombinzeit nach Eintrag von Polymerwerkstoff mit medikamenthöser Wirkung in Humanzitratplasma | |
|---|---|---|
| Inkubationszeit (min) | Verlängerung der Trombinzeit | |
| | sec. | % |
| 0 | 28 | 187 |
| 30 | 34 | 227 |
| 60 | 38 | 253 |
| 120 | 40 | 267 |
| 180 | 41 | 273 |
| 240 | 44 | 293 |
| 300 | 49 | 327 |

Diese Werte wurden mit der direkten Injektion von Heparin verglichen, die Heparin-Thrombinzeit betrug bei 15 sec. 100%.

Es zeigt sich, daß das Polymermaterial mit medikamentöser Wirkung gemäß der vorliegenden Erfindung im Vergleich mit dem konventionellen Verfahren der Injektion zu einer wesentlichen Verlängerung der Thrombinzeit führte.

## Patentansprüche

1. Verfahren zur Herstellung von Erzeugnissen aus Polymerwerkstoffen mit medikamentöser Depotwirkung, mit folgenden Schritten:
- Vermischen der Ausgangsmaterialien
- Formen der Mischung in eine gewünschte Form,
gekennzeichnet, durch
- Einbringen der so hergestellten Form in eine Schutzverpackung, und
- Vernetzen und Sterilisieren der Form in der Schutzverpackung,

2. Verfahren gemäß Anspruch 1
**dadurch gekennzeichnet,** daß die Vernetzung und Sterilisierung durch Elektronenstrahlung erfolgt.

3. Verfahren nach mindestens einem der Ansprüche 1 oder 2,
**dadurch gekennzeichnet,** daß die Vernetzung und Sterilisierung mit Strahlendosen von 10 bis 120 KGy durchgeführt wird.

4. Verfahren nach wenigstens einem der Ansprüche 1 oder 2,
**dadurch gekennzeichnet,** daß die Vernetzung und Sterilisierung mit Strahlendosen von 20 bis 66 KGy durchgeführt wird.

5. Verfahren nach mindestens einem der Ansprüche 1 bis 4,
**dadurch gekennzeichnet**, daß dem Polymerwerkstoff Substanzen zugegeben werden, die als Aktivatoren sowie als Vernetzer wirken, um die Strahlendosis zu verringern.

6. Verfahren nach Anspruch 5,
**dadurch gekennzeichnet,** daß als aktivierend und vernetzend wirkende Substanz Acrylate oder deren Derivate zugegeben werden.

7. Verfahren nach Anspruch 5 oder 6,
**dadurch gekennzeichnet,** daß als aktivierend und vernetzend wirkende Substanz Trimethylolpropantrimethacrylat zugegeben wird.

8. Verfahren nach mindestens einen der Ansprüche 5 bis 7,
**dadurch gekennzeichnet,** daß die als aktivierend und vernetzend wirkende Substanz in einer Menge von 0 bis 20 Gew.% zugegeben wird.

9. Verfahren nach einen der Ansprüche 5 bis 7,
**dadurch gekennzeichnet,** daß die als aktivierend und vernetzend wirkende Substanz in einer Menge von 0 bis 5 Gew.% zugegeben wird.

10. Verfahren nach mindestens einem der Ansprüche 1 bis 9,
**dadurch gekennzeichnet,** daß als Polymerwerkstoffe natürliche und/oder synthetische Polymere verwendet werden.

11. Verfahren nach mindestens einem der Ansprüche 1 bis 10,
**dadurch gekennzeichnet,** daß dem Polymerwerkstoff eine biologisch aktive Substanz zugegeben wird.

12. Verfahren nach Anspruch 11,
**dadurch gekennzeichnet,** daß die biologisch aktive Substanz vor dem Vermischen mit dem Polymerwerkstoff an eine anorganische oder organische Trägersubstanz gebunden wird.

13. Verfahren nach Anspruch 12,
**dadurch gekennzeichnet,** daß als Trägersubstanz Molsiebe und/oder Schichtsilikate verwendet werden.

14. Verfahren nach Anspruch 11 oder 12,
**dadurch gekennzeichnet,** daß als biologisch aktive Substanz hoch- und/oder niedermolekulare Heparine oder Heparinoide verwendet werden.

15. Verfahren nach mindestens einem der Ansprüche 1-14,
**dadurch gekennzeichnet,** daß der Polymerwerkstoff nach dem Einbringen in die Schutzverpackung und vor dem Vernetzen und Sterilisieren in eine wärmeleitende Form gelegt wird.

16. Verfahren nach Anspruch 15,
**dadurch gekennzeichnet**, daß als wärmeleitende Form eine Metallform eingesetzt wird.

17. Verfahren nach mindestens einem der Ansprüche 1 bis 16,
**dadurch gekennzeichnet,** daß die verwendeten Polymerwerkstoffe natürliche oder synthetische Polymere in einer Menge von 20 bis 80 Gew.%, Kreide oder andere geeignete natürliche oder synthetische Füllstoffe in einer Menge von 0 bis 80 Gew.%, Vernetzungsaktivatoren in einer Menge von 0 bis 5 Gew.% und ein Trägersystem/Medikament-Addukt in einer Menge von 1 bis 50 Gew.% umfassen.

18. Verfahren nach Anspruch 17, **dadurch gekennzeichnet**, daß zusätzlich geeignete Farbstoffe und in der Polymerverarbeitung übliche Zusatzstoffe verwendet werden.

19. Verfahren nach mindestens einem der Ansprüche 17 oder 18, **gekennzeichnet durch** folgende Zusammensetzung der Ausgangsmaterialien: Naturkautschuk in einer Menge von 37,17%, Kreide in einer Menge von 46,47%, Vernetzungsaktivator in einer Menge von 1,49% und Molsieb/Medikament-Addukt in einer Menge von 14,87%.

20. Verfahren nach mindestens einem der Ansprüche 17 oder 18, **gekennzeichnet durch** folgende Zusammensetzungen der Ausgangsmaterialen: Siliconkautschuk in einer Menge von 52,6%, Kreide in einer Menge von 31,6% und Molsieo/Medikament-Addukt in einer Menge von 15,8%.

## Claims

1. Method of producing products from polymer materials with a medicamentous depot effect, comprising the following steps:
- mixing the starting materials,
- shaping the mixture into a desired mold,
**characterized by**
- introducing the resultant mold into a protective packaging, and
- curing and sterilizing said mold in said protective packaging.

2. The method according to claim 1,
**characterized in** that curing and sterilization are performed through electron radiation.

3. The method according to at least one of claims 1 or 2,
**characterized in** that curing and sterilization are performed with radiation doses of from 10 to 120 KGy.

4. The method according to at least one of claims 1 or 2,
**characterized in** that curing and sterilization are performed with radiation doses of from 20 to 66 KGy.

5. The method according to at least one of claims 1 to 4,
**characterized in** that substances which act as activators and curing agents are added to said polymer material for reducing the radiation dose.

6. The method according to claim 5,
**characterized in** that acrylates or the derivatives thereof are added as the substance having an activating and curing effect.

7. The method according to claim 5 or 6,
**characterized in** that trimethylolpropane trimethacrylate is added as said substance having an activating and curing effect.

8. The method according to at least one of claims 5 to 7,
**characterized in** that said substance with said activating and curing effect is added in an amount of from 0 to 20% by weight.

9. The method according to any of claims 5 to 7,
**characterized in** that said substance with said activating and curing effect is added in an amount of from 0 to 5% by weight.

10. The method according to at least one of claims 1 to 9,
**characterized in** that natural and/or synthetic polymers are used as said polymer materials.

11. The method according to at least one of claims 1 to 10,
**characterized in** that a biologically active substance is added to said polymer material.

12. The method according to claim 11,
**characterized in** that said biologically active substance is bound to an inorganic or organic carrier prior to mixing with said polymer material.

13. The method according to claim 12,
**characterized in** that molecular sieves and/or layered silicates are used as said carrier.

14. The method according to claim 11 or 12,
**characterized in** that high- and/or low-molecular heparins or heparinoids are used as said biologically active substance.

15. The method according to at least one of claims 1 to 14,
**characterized in** that said polymer material is put into a heat-conducting mold after insertion into said protective packaging and prior to curing and sterilization.

16. The method according to claim 15,
**characterized in** that a metal mold is used as said heat-conducting mold.

17. The method according to at least one of claims 1 to 16,
**characterized in** that the used polymer materials comprise natural or synthetic polymers in an amount of from 20 to 80% by weight, chalk or other suitable natural or synthetic fillers in an amount of from 0 to 80% by weight, curing activators in an amount of from 0 to 5% by weight and a carrier system/medicament adduct in an amount of from 1 to 50% by weight.

18. The method according to claim 17,
**characterized in** that suitable dyes as well as additives which are standard in polymer processing are additionally used.

19. The method according to at least one of claims 17 or 18,
**characterized by** the following composition of the starting materials: natural rubber in an amount of 37.17%, chalk in an amount of 46.47%, curing activators in an amount of 1.49% and molecular sieve/medicament adduct in an amount of 14.87%.

## Revendications

1. Procédé de fabrication de produits en matériaux polymères à effet médicamenteux retard, avec les étapes suivantes consistant à :
• mélanger les matières premières
• façonner le mélange dans une forme souhaitée,
caractérisé par les étapes consistant à
• introduire la forme ainsi fabriquée dans un emballage de protection, et
• réticuler et stériliser la forme dans son emballage de protection.

2. Procédé selon la revendication 1, caractérisé par le fait que la réticulation et la stérilisation se font par rayonnement électronique.

3. Procédé selon au moins l'une des revendications 1 et 2, caractérisé par le fait que la réticulation et la stérilisation sont réalisées avec des doses de rayonnement de 10 à 120 KGy.

4. Procédé selon au moins l'une des revendications 1 et 2, caractérisé par le fait que la réticulation et la stérilisation sont réalisées avec des doses de rayonnement de 20 à 66 KGy.

5. Procédé selon au moins l'une des revendications 1 à 4, caractérisé par le fait que l'on ajoute au matériau polymère des substances qui sont efficaces comme activateurs tout comme agents de réticulation pour réduire la dose de rayonnement.

6. Procédé selon la revendication 5, caractérisé par le fait que l'on ajoute comme substance à effet activant et réticulant des acrylates ou des dérivés d'acrylate.

7. Procédé selon la revendication 5 ou 6, caractérisé par le fait que l'on ajoute comme substance à effet activant et réticulant du triméthylacrylate de triméthylolpropane.

8. Procédé selon au moins l'une des revendications 5 à 7, caractérisé par le fait que l'on ajoute la substance à effet activant et réticulant en une quantité de 0 à 20 % en poids.

9. Procédé selon l'une des revendications 5 à 7, caractérisé par le fait que l'on ajoute la substance à effet activant et réticulant en une quantité de 0 à 5% en poids.

10. Procédé selon au moins l'une des revendications 1 à 9, caractérisé par le fait que l'on utilise des polymères naturels et/ou synthétiques comme matériaux polymères.

11. Procédé selon au moins l'une des revendications 1 à 10, caractérisé par le fait que l'on ajoute au matériau polymère une substance biologiquement active.

12. Procédé selon la revendication 11, caractérisé par le fait que la substance biologiquement active est combinée avec une substance support, minérale ou organique, avant le mélange avec le matériau polymère.

13. Procédé selon la revendication 12, caractérisé par le fait que l'on utilise comme substance support des tamis moléculaires et/ou des silicates stratifiés.

14. Procédé selon la revendication 11 ou 12, caractérisé par le fait que l'on utilise comme substance biologiquement active de l'héparine à fort et/ou faible poids moléculaire ou de l'héparinoide.

15. Procédé selon au moins l'une des revendications 1 à 14, caractérisé par le fait que le matériau polymère est placé dans une forme conduisant la chaleur après introduction dans l'emballage de protection et avant la réticulation et la stérilisation.

16. Procédé selon la revendication 15, caractérisé par le fait que l'on emploie pour la forme conduisant la chaleur une forme métallique.

17. Procédé selon au moins l'une des revendications 1 à 16, caractérisé par le fait que les matériaux polymères utilisés contiennent des polymères naturels ou synthétiques en une quantité de 20 à 80% en poids, de la craie ou d'autres charges appropriées, naturelles ou synthétiques en une quantité de 0 à 80% en poids, des activateurs de réticulation en une quantité de 0 à 5% en poids et un produit d'addition système support/médicament en une quantité de 1 à 50% en poids.

18. Procédé selon la revendication 17, caractérisé par le fait que l'on utilise en supplément des colorants appropriés et pendant le traitement du polymère des additifs appropriés.

19. Procédé selon au moins l'une des revendications 17 et 18, caractérisé par la composition suivante des matières premières : caoutchouc naturel en une quantité de 37,17%, craie en une quantité de 46,47%, activateur de réticulation en une quantité de 1,49% et produit d'addition tamis moléculaire/médicament en une quantité de 14,87%.

20. Procédé selon au moins l'une des revendications 17 et 18, caractérisé par la composition suivante des matières premières : caoutchouc de silicone en une quantité de 52,6%, craie en une quantité de 31,6% et produit d'addition tamis moléculaire/médicament en une quantité de 15,8%.
